# EUROPÄISCHE PATENTSCHRIFT

(11) **EP 3 827 031 B1**
(45) Veröffentlichungstag und Bekanntmachung des Hinweises auf die Patenterteilung: **26.02.2025**
(21) Anmeldenummer: 19737765.8
(22) Anmeldetag: 15.07.2019
(51) Int. Cl.: C08J 3/12, C08J 3/075, C08J 3/24, A61L 15/22, A61L 15/60, C08F 6/00

(54) **VERFAHREN ZUR HERSTELLUNG VON SUPERABSORBERN**
METHOD FOR THE PRODUCTION OF SUPERABSORBERS
PROCÉDÉ DE FABRICATION DE SUPERABSORBANTS

(30) Priorität: 24.07.2018 EP 18185245
(43) Veröffentlichungstag der Anmeldung: 02.06.2021
(73) Patentinhaber: BASF SE, 67056 Ludwigshafen am Rhein (DE)
(72) Erfinder: KRUEGER, Marco, 67056 Ludwigshafen (DE); FUNK, Ruediger, 67056 Ludwigshafen (DE); PFEIFFER, Thomas, 67056 Ludwigshafen (DE); POSSEMIERS, Karl, 2040 Antwerpen (BE); SCHROEDER, Juergen, 67056 Ludwigshafen (DE); WEISMANTEL, Matthias, 67056 Ludwigshafen (DE)
(74) Vertreter: BASF IP Association
(86) Internationale Anmeldenummer: PCT/EP2019/068942
(87) Internationale Veröffentlichungsnummer: WO 2020/020675

(56) Entgegenhaltungen:
- DE-A1- 102005 042 604
- DE-A1- 102005 042 605
- DE-A1- 102005 042 606

## Beschreibung

Die vorliegende Erfindung betrifft ein Verfahren zur Herstellung von Superabsorberpartikeln durch Polymerisation einer Monomerlösung oder -suspension, umfassend die Schritte kontinuierliche Vorneutralisation eines Monomeren unter Kühlung und teilweiser Rückführung des vorneutralisierten Monomeren in die Vorneutralisation, kontinuierliche Erwärmung des vorneutralisierten Monomeren mittels eines Wärmetauschers und kontinuierliche Nachneutralisation des erwärmten Monomeren, wobei die Monomerlösung oder -suspension vor dem Eintritt in den Polymerisationsreaktor eine Temperatur von 70 bis 99°C aufweist und die Temperatur des im Wärmetauscher verwendeten Heizmediums weniger als 160°C beträgt.

Superabsorber werden zur Herstellung von Windeln, Tampons, Damenbinden und anderen Hygieneartikeln, aber auch als wasserzurückhaltende Mittel im landwirtschaftlichen Gartenbau verwendet. Die Superabsorber werden auch als wasserabsorbierende Polymere bezeichnet.

Die Herstellung von Superabsorbern wird in der Monographie "Modern Superabsorbent Polymer Technology", F.L. Buchholz und A.T. Graham, Wiley-VCH, 1998, Seiten 71 bis 103, beschrieben.

Die Eigenschaften der Superabsorber können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften, wie beispielsweise Gelbettpermeabilität (GBP) und Absorption unter einem Druck von 49,2 g/cm² (AUL0.7psi), werden Superabsorberpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächen nachvernetzt. Dazu geeignete Vernetzer sind Verbindungen, die mit mindestens zwei Carboxylatgruppen der Polymerpartikel kovalente Bindungen bilden können.

US 2011/0313113 und US 2012/0258851 offenbaren die Polymerisation von Monomerlösungen mit dispergierten Gasblasen. Als eine Möglichkeit zur Erzeugung der dispergierten Gasblasen wird der Start der Polymerisation bei sehr hohen Temperaturen erwähnt.

Aufgabe der vorliegenden Erfindung war die Bereitstellung eines verbesserten Verfahrens zur Herstellung von Superabsorbern, insbesondere eine Verringerung der Verweilzeit der Monomerlösung bei sehr hohen Temperaturen vor dem Eintritt in den Polymerisationsreaktor bei gleichzeitig sehr hoher Temperatur der Monomerlösung bei Eintritt in den Polymerisationsreaktor.

Gelöst wurde die Aufgabe durch ein Verfahren zur Herstellung von Superabsorbern durch Polymerisation einer Monomerlösung oder -suspension, enthaltend
a) mindestens ein ethylenisch ungesättigtes, säuregruppentragendes Monomer, das zumindest teilweise neutralisiert ist,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

umfassend die Schritte
   i) kontinuierliche Vorneutralisation des Monomeren unter Kühlung und teilweiser Rückführung des vorneutralisierten Monomeren in die Vorneutralisation,
   ii) kontinuierliche Erwärmung des vorneutralisierten Monomeren aus Schritt i) mittels eines Wärmetauschers,
   iii) kontinuierliche Nachneutralisation des erwärmten Monomeren aus Schritt ii),
   iv) Zusatz des Vernetzers zur Monomerlösung aus Schritt iii) und
   v) Zusatz des Initiators zur Monomerlösung aus Schritt iv),
wobei die Monomerlösung oder -suspension vor dem Eintritt in den Polymerisationsreaktor eine Temperatur von 70 bis 99°C aufweist und die Temperatur des im Wärmetauscher verwendeten Heizmediums in Schritt ii) weniger als 160°C beträgt.

Das vorneutralisierte Monomer wird in Schritt i) auf eine Temperatur von vorzugsweise weniger als 45°C, besonders bevorzugt weniger als 40°C, ganz besonders bevorzugt weniger als 35°C, gekühlt. Niedrigere Temperaturen senken die Polymerisationsneigung.

Das vorneutralisierte Monomer wird vorzugsweise zu 40 bis 98%, besonders bevorzugt zu 60 bis 95%, ganz besonders bevorzugt zu 80 bis 90%, in Schritt i) rückgeführt. Durch die Rückführung wird frisches Monomer mit vorneutralisierter Monomerlösung verdünnt und Überhitzungen vermieden.

Ein geeignetes Verfahren zur kontinuierlichen Vorneutralisation mit Rückführung wird beispielsweise in der WO 2007/028751 A2 beschrieben.

Die Temperatur des Heizmediums in Schritt ii) beträgt vorzugsweise weniger als 140°C, besonders bevorzugt weniger als 120°C, ganz besonders bevorzugt weniger als 110°C.

In der Nachneutralisation in Schritt iii) wird der Neutralisationsgrad des Monomeren a) um vorzugsweise 5 bis 45 mol-%, besonders bevorzugt 10 bis 35 mol-%, ganz besonders bevorzugt 15 bis 25 mol-%, erhöht.

Ein geeignetes Verfahren zur kontinuierlichen Nachneutralisation wird beispielsweise in der WO 2007/028747 A1 beschrieben.

Der vorliegenden Erfindung liegt die Erkenntnis zugrunde, dass die Verweilzeit der Monomerlösung bei sehr hohen Temperaturen vor dem Eintritt in den Polymerisationsreaktor durch geeignete Reaktionsführung und damit die Polymerisationsneigung vor dem Eintritt in den Polymerisationsreaktor gesenkt werden kann.

In einer besonders bevorzugten Ausführungsform der vorliegenden Erfindung wird der Monomerlösung vor Schritt iii) ein Chelatbildner zugesetzt.

In einer ganz besonders bevorzugten Ausführungsform der vorliegenden Erfindung wird der Monomerlösung vor Schritt ii) ein Chelatbildner zugesetzt.

Im Folgenden wird die Herstellung der Superabsorber näher erläutert:
Die Superabsorber werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 02/055469 A1, der WO 03/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di-oder Triacrylaten veresterten mehrfach ethoxylierten und/oder propoxylierten Glyzerine, wie sie beispielsweise in WO 03/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 0,8 Gew.-%, ganz besonders bevorzugt 0,15 bis 0,5 Gew.-%, jeweils berechnet auf die Gesamtmenge an eingesetztem Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird vorzugsweise das Dinatriumsalz der 2-Hydroxy-2-sulfonatoessig-säure oder ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite^{®} FF6 und Brüggolite^{®} FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit der Löslichkeit überschreitendem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren für die Polymerisation sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 85 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen. Feste Carbonate und Hydrogencarbonate können hierbei auch in verkapselter Form eingesetzt werden, vorzugsweise in die Monomerlösung direkt vor der Polymerisation, während oder nach der Polymerisation ins Polymergel und vor dessen Trocknung. Die Verkapselung erfolgt durch Beschichtung der Oberfläche mit einem unlöslichen oder nur langsam löslichen Material (beispielsweise mittels filmbildender Polymere, inerter anorganischer Materialien oder schmelzbaren organischer Materialien), welches die Lösung und Reaktion des festen Carbonats oder Hydrogencarbonats so verzögert, dass erst während der Trocknung Kohlendioxid freigesetzt wird und der entstehende Superabsorber eine hohe innere Porosität aufweist.

Optional kann zur Monomerlösung vor oder während der Polymerisation ein Tensid zugegeben werden und die Monomerlösung dann vor oder während der Polymerisation mit einem Inertgas oder Wasserdampf oder durch starkes Rühren geschäumt werden. Das Tensid kann anionisch, kationisch, zwitterionisch oder auch nicht-ionisch sein. Bevorzugt wird ein hautfreundliches Tensid eingesetzt.

Das Polymergel wird dann in üblicherweise mit einem Umluftbandtrockner getrocknet bis der Restfeuchtegehalt vorzugsweise 0,5 bis 10 Gew.-%, besonders bevorzugt 1 bis 6 Gew.-%, ganz besonders bevorzugt 1,5 bis 4 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Polymergels beträgt vor der Trocknung vorzugsweise von 25 und 90 Gew.-%, besonders bevorzugt von 35 bis 70 Gew.-%, ganz besonders bevorzugt von 40 bis 60 Gew.-%. Anschließend wird das getrocknete Polymergel gebrochen und optional grob zerkleinert.

Das getrocknete Polymergel wird hiernach üblicherweise gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise von 150 bis 850 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Polymerpartikeln mit einer Partikelgröße von größer 150 µm beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Gelbettpermeabilität (GBP). Daher sollte der Anteil zu kleiner Polymerpartikel ("fines") niedrig sein.

Zu kleine Polymerpartikel werden daher üblicherweise abgetrennt und in das Verfahren rückgeführt, vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt. Es ist aber auch möglich die zu kleinen Polymerpartikel in einem dem Polymerisationsreaktor nachgeschalteten Kneter oder Extruder in das Polymergel einzuarbeiten.

Werden die zu kleinen Polymerpartikel sehr früh zugesetzt, beispielsweise bereits zur Monomerlösung, so wird dadurch die Zentrifugenretentionskapazität (CRC) der erhaltenen Polymerpartikel gesenkt. Dies kann aber beispielsweise durch Anpassung der Einsatzmenge an Vernetzer b) kompensiert werden.

Der Anteil an Polymerpartikeln mit einer Partikelgröße von höchstens 850 µm, beträgt vorzugsweise mindestens 90 Gew.-%, besonders bevorzugt mindestens 95 Gew.-%, ganz besonders bevorzugt mindestens 98 Gew.-%.

Der Anteil an Polymerpartikeln mit einer Partikelgröße von höchstens 600 µm, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Quellgeschwindigkeit. Daher sollte der Anteil zu großer Polymerpartikel ebenfalls niedrig sein. Zu große Polymerpartikel werden daher üblicherweise abgetrennt und in die Mahlung rückgeführt.

Die Polymerpartikel können zur weiteren Verbesserung der Eigenschaften thermisch oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des Weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 03/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 3 Gew.-%, besonders bevorzugt 0,02 bis 1 Gew.-%, ganz besonders bevorzugt 0,05 bis 0,2 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar^{®} Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix^{®} (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex^{®} Horizontal Paddle Dryer (Hosokawa Micron GmbH; Leingarten; Deutschland), Hosokawa Bepex^{®} Disc Dryer (Hosokawa Micron GmbH; Leingarten; Deutschland), Holo-Flite^{®} dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächennachvernetzt.

Bevorzugte Reaktionstemperaturen liegen im Bereich 100 bis 250°C, bevorzugt 110 bis 220°C, besonders bevorzugt 120 bis 210°C, ganz besonders bevorzugt 130 bis 200°C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die Polymerpartikel nach der Oberflächennachvernetzung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex^{®} Horizontal Paddle Cooler (Hosokawa Micron GmbH; Leingarten; Deutschland), Hosokawa Bepex^{®} Disc Cooler (Hosokawa Micron GmbH; Leingarten; Deutschland), Holo-Flite^{®} coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die Polymerpartikel auf vorzugsweise 40 bis 90°C, besonders bevorzugt 45 bis 80°C, ganz besonders bevorzugt 50 bis 70°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 40 bis 120°C, besonders bevorzugt bei 50 bis 110°C, ganz besonders bevorzugt bei 60 bis 100°C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Oberflächennachvernetzung durchgeführt. Geeignete Beschichtungen zur Verbesserung der Quellgeschwindigkeit sowie der Gelbettpermeabilität (GBP) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil^{®} 200, und Tenside, wie Span^{®} 20. Geeignete Beschichtungen zur Staubbindung, zur Verringerung der Verbackungsneigung sowie zur Erhöhung der mechanischen Stabilität sind Polymerdispersionen, wie in EP 0 703 265 B1 beschrieben, und Wachse, wie in US 5 840 321 beschrieben.

Anschließend können die beschichteten und/oder nachbefeuchteten Polymerpartikel erneut klassiert werden, wobei zu kleine und/oder zu große Polymerpartikel abgetrennt und in das Verfahren rückgeführt werden.

### Beispiele

Die Beispiele wurden mittels fachüblicher Simulationsprogramme berechnet.

### Beispiel 1

In einer kontinuierlichen Vorneutralisation werden 5715 kg/h (79,3 kMol/h) Acrylsäure, 3635 kg/h (45,4 kMol/h) 50 gew.-%ige Natronlauge und 5650 kg/h Wasser gemischt und mittels eines Rohrbündelwärmetauschers auf 30°C gekühlt. In die Vorneutralisation werden 85% der gekühlten Mischung zurückgeführt. Der Neutralisationsgrad beträgt 57,3 mol-%. Der Feststoffgehalt beträgt 44,8 Gew.-%. Die Verweilzeit der Monomerlösung in der Vorneutralisation beträgt ca. eine Stunde.

Mittels eines Rohrbündelwärmetauschers werden 15000 kg/h der vorneutralisierten Monomerlösung im Gegenstrom erwärmt. Der Rohrbündelwärmetauscher hat eine innere Oberfläche von 35 m². Die Temperatur des Heizmediums (Vorlauftemperatur) beträgt 100°C (Kondensat). Die Menge an Heizmedium beträgt 12100 kg/h. Die Temperatur des Heizmediums beträgt am Ausgang des Wärmetauschers 60°C. Die Heizleistung des Wärmetauschers beträgt 562 kW. Die Temperatur der Monomerlösung steigt auf 75°C. Die Verweilzeit der Monomerlösung im Rohrbündelwärmetauscher beträgt ca. 2,5 Minuten.

Die erwärmte Monomerlösung wird mit 1123 kg/h (14,0 kMol/h) 50 gew.-%ige Natronlauge nachneutralisiert. Die Temperatur der Natronlauge beträgt 40°C. Durch die Neutralisationswärme steigt die Temperatur auf 95°C. Der Neutralisationsgrad beträgt 75,0 mol-%. Der Feststoffgehalt beträgt 43,6 Gew.-%. Die Verweilzeit der Monomerlösung in der Nachneutralisation beträgt ca. 12 Sekunden.

Anschließend werden der nachneutralisierten Monomerlösung nacheinander Vernetzer und Initiator zugesetzt, die Monomerlösung in deinen Polymerisationsreaktor überführt und polymerisiert.

### Beispiel 2 (Vergleichsbeispiel)

In einer kontinuierlichen Vorneutralisation werden 5715 kg/h (79,3 kMol/h) Acrylsäure, 3635 kg/h (45,4 kMol/h) 50 gew.-%ige Natronlauge und 5650 kg/h Wasser gemischt und mittels eines Rohrbündelwärmetauschers auf 30°C gekühlt. In die Vorneutralisation werden 85% der gekühlten Mischung zurückgeführt. Der Neutralisationsgrad beträgt 57,3 mol-%. Der Feststoffgehalt beträgt 44,8 Gew.-%. Die Verweilzeit der Monomerlösung in der Vorneutralisation beträgt ca. eine Stunde.

15000 kg/h der vorneutralisierten Monomerlösung werden mit 1123 kg/h (14,0 kMol/h) 50 gew.-%ige Natronlauge nachneutralisiert. Die Temperatur der Natronlauge beträgt 40°C. Durch die Neutralisationswärme steigt die Temperatur auf 53,4°C. Der Neutralisationsgrad beträgt 75,0 mol-%. Der Feststoffgehalt beträgt 43,6 Gew.-%. Die Verweilzeit der Monomerlösung in der Nachneutralisation beträgt ca. 12 Sekunden.

Mittels eines Rohrbündelwärmetauschers wird die nachneutralisierte Monomerlösung erwärmt. Der Rohrbündelwärmetauscher hat eine innere Oberfläche von 35 m². Die Temperatur des Heizmediums (Vorlauftemperatur) beträgt 100°C (Kondensat). Die Menge an Heizmedium beträgt 12100 kg/h. Die Temperatur des Heizmediums beträgt am Ausgang des Wärmetauschers 73°C. Die Heizleistung des Wärmetauschers beträgt 378 kW. Die Temperatur der Monomerlösung steigt auf 83,7°C. Die Verweilzeit der Monomerlösung im Rohrbündelwärmetauscher beträgt ca. 2,5 Minuten.

Anschließend werden der erwärmten Monomerlösung nacheinander Vernetzer und Initiator zugesetzt, die Monomerlösung in deinen Polymerisationsreaktor überführt und polymerisiert.

### Beispiel 3 (Vergleichsbeispiel)

In einer kontinuierlichen Vorneutralisation werden 5715 kg/h (79,3 kMol/h) Acrylsäure, 3635 kg/h (45,4 kMol/h) 50 gew.-%ige Natronlauge und 5650 kg/h Wasser gemischt und mittels eines Rohrbündelwärmetauschers auf 30°C gekühlt. In die Vorneutralisation werden 85% der gekühlten Mischung zurückgeführt. Der Neutralisationsgrad beträgt 57,3 mol-%. Der Feststoffgehalt beträgt 44,8 Gew.-%. Die Verweilzeit der Monomerlösung in der Vorneutralisation beträgt ca. eine Stunde.

15000 kg/h der vorneutralisierten Monomerlösung werden mit 1123 kg/h (14,0 kMol/h) 50 gew.-%ige Natronlauge nachneutralisiert. Die Temperatur der Natronlauge beträgt 40°C. Durch die Neutralisationswärme steigt die Temperatur auf 53,4°C. Der Neutralisationsgrad beträgt 75,0 mol-%. Der Feststoffgehalt beträgt 43,6 Gew.-%. Die Verweilzeit der Monomerlösung in der Nachneutralisation beträgt ca. 12 Sekunden.

Mittels eines Rohrbündelwärmetauschers wird die nachneutralisierte Monomerlösung erwärmt. Der Rohrbündelwärmetauscher hat eine innere Oberfläche von 35 m². Die Temperatur des Heizmediums (Vorlauftemperatur) beträgt 111°C (1,5 bar Heizdampf). Die Menge an Heizmedium beträgt 897 kg/h. Die Temperatur des Heizmediums beträgt am Ausgang des Wärmetauschers 60°C. Die Heizleistung des Wärmetauschers beträgt 520 kW. Die Temperatur der Monomerlösung steigt auf 95°C. Die Verweilzeit der Monomerlösung im Rohrbündelwärmetauscher beträgt ca. 2,5 Minuten.

Anschließend werden der erwärmten Monomerlösung nacheinander Vernetzer und Initiator zugesetzt, die Monomerlösung in deinen Polymerisationsreaktor überführt und polymerisiert.

### Beispiel 4

In einer kontinuierlichen Vorneutralisation werden 5715 kg/h (79,3 kMol/h) Acrylsäure, 3635 kg/h (45,4 kMol/h) 50 gew.-%ige Natronlauge und 5650 kg/h Wasser gemischt und mittels eines Rohrbündelwärmetauschers auf 30°C gekühlt. In die Vorneutralisation werden 85% der gekühlten Mischung zurückgeführt. Der Neutralisationsgrad beträgt 57,3 mol-%. Der Feststoffgehalt beträgt 44,8 Gew.-%. Die Verweilzeit der Monomerlösung in der Vorneutralisation beträgt ca. eine Stunde.

Mittels eine Rohrbündelwärmetauschers werden 15000 kg/h der vorneutralisierten Monomerlösung im Gegenstrom erwärmt. Der Rohrbündelwärmetauscher hat eine innere Oberfläche von 9,4 m². Die Temperatur des Heizmediums (Vorlauftemperatur) beträgt 150°C (4,8 bar Heizdampf). Die Menge an Heizmedium beträgt 960 kg/h. Die Temperatur des Heizmediums beträgt am Ausgang des Wärmetauschers 60°C. Die Heizleistung des Wärmetauschers beträgt 562 kW. Die Temperatur der Monomerlösung steigt auf 75°C. Die Verweilzeit der Monomerlösung im Rohrbündelwärmetauscher beträgt ca. 40 Sekunden.

Die erwärmte Monomerlösung wird mit 1123 kg/h (14,0 kMol/h) 50 gew.-%ige Natronlauge nachneutralisiert. Die Temperatur der Natronlauge beträgt 40°C. Durch die Neutralisationswärme steigt die Temperatur auf 95°C. Der Neutralisationsgrad beträgt 75,0 mol-%. Der Feststoffgehalt beträgt 43,6 Gew.-%. Die Verweilzeit der Monomerlösung in der Nachneutralisation beträgt ca. 12 Sekunden.

Anschließend werden der nachneutralisierten Monomerlösung nacheinander Vernetzer und Initiator zugesetzt, die Monomerlösung in deinen Polymerisationsreaktor überführt und polymerisiert.

### Beispiel 5 (Vergleichsbeispiel)

In einer kontinuierlichen Vorneutralisation werden 5715 kg/h (79,3 kMol/h) Acrylsäure, 3635 kg/h (45,4 kMol/h) 50 gew.-%ige Natronlauge und 5650 kg/h Wasser gemischt und mittels eines Rohrbündelwärmetauschers auf 30°C gekühlt. In die Vorneutralisation werden 85% der gekühlten Mischung zurückgeführt. Der Neutralisationsgrad beträgt 57,3 mol-%. Der Feststoffgehalt beträgt 44,8 Gew.-%. Die Verweilzeit der Monomerlösung in der Vorneutralisation beträgt ca. eine Stunde.

15000 kg/h der vorneutralisierten Monomerlösung werden mit 1123 kg/h (14,0 kMol/h) 50 gew.-%ige Natronlauge nachneutralisiert. Die Temperatur der Natronlauge beträgt 40°C. Durch die Neutralisationswärme steigt die Temperatur auf 53,4°C. Der Neutralisationsgrad beträgt 75,0 mol-%. Der Feststoffgehalt beträgt 43,6 Gew.-%. Die Verweilzeit der Monomerlösung in der Nauchneutralisation beträgt ca. 12 Sekunden.

Mittels eine Rohrbündelwärmetauschers wird die nachneutralisierte Monomerlösung erwärmt. Der Rohrbündelwärmetauscher hat eine innere Oberfläche von 9,4 m². Die Temperatur des Heizmediums (Vorlauftemperatur) beträgt 150°C (4,8 bar Heizdampf). Die Menge an Heizmedium beträgt 960 kg/h. Die Temperatur des Heizmediums beträgt am Ausgang des Wärmetauschers 60°C. Die Heizleistung des Wärmetauschers beträgt 446 kW. Die Temperatur der Monomerlösung steigt auf 89°C. Die Verweilzeit der Monomerlösung im Rohrbündelwärmetauscher beträgt ca. 40 Sekunden.

Anschließend werden der erwärmten Monomerlösung nacheinander Vernetzer und Initiator zugesetzt, die Monomerlösung in deinen Polymerisationsreaktor überführt und polymerisiert.

### Beispiel 6 (Vergleichsbeispiel)

In einer kontinuierlichen Vorneutralisation werden 5715 kg/h (79,3 kMol/h) Acrylsäure, 3635 kg/h (45,4 kMol/h) 50 gew.-%ige Natronlauge und 5650 kg/h Wasser gemischt und mittels eines Rohrbündelwärmetauschers auf 30°C gekühlt. In die Vorneutralisation werden 85% der gekühlten Mischung zurückgeführt. Der Neutralisationsgrad beträgt 57,3 mol-%. Der Feststoffgehalt beträgt 44,8 Gew.-%. Die Verweilzeit der Monomerlösung in der Vorneutralisation beträgt ca. eine Stunde.

15000 kg/h der vorneutralisierten Monomerlösung werden mit 1123 kg/h (14,0 kMol/h) 50 gew.-%ige Natronlauge nachneutralisiert. Die Temperatur der Natronlauge beträgt 40°C. Durch die Neutralisationswärme steigt die Temperatur auf 53,4°C. Der Neutralisationsgrad beträgt 75,0 mol-%. Der Feststoffgehalt beträgt 43,6 Gew.-%. Die Verweilzeit der Monomerlösung in der Nauchneutralisation beträgt ca. 12 Sekunden.

Mittels eine Rohrbündelwärmetauschers wird die nachneutralisierte Monomerlösung erwärmt. Der Rohrbündelwärmetauscher hat eine innere Oberfläche von 9,4 m². Die Temperatur des Heizmediums (Vorlauftemperatur) beträgt 165°C (7 bar Heizdampf). Die Menge an Heizmedium beträgt 920 kg/h. Die Temperatur des Heizmediums beträgt am Ausgang des Wärmetauschers 60°C. Die Heizleistung des Wärmetauschers beträgt 520 kW. Die Temperatur der Monomerlösung steigt auf 95°C. Die Verweilzeit der Monomerlösung im Rohrbündelwärmetauscher beträgt ca. 40 Sekunden.

Anschließend werden der erwärmten Monomerlösung nacheinander Vernetzer und Initiator zugesetzt, die Monomerlösung in deinen Polymerisationsreaktor überführt und polymerisiert.

Die Beispiele zeigen, dass das erfindungsgemäße Verfahren niedrigere Vorlauftemperaturen im Wärmetauscher ermöglicht. Höhere Vorlauftemperaturen erhöhen aber die Verschmutzung des Wärmetauschers durch Polymerablagerungen. Außerdem wird dadurch das erfindungsgemäße Verfahren die Verweilzeit bei höheren Temperaturen gesenkt.

## Patentansprüche

1. Verfahren zur Herstellung von Superabsorberpartikeln durch Polymerisation einer Monomerlösung oder -suspension, enthaltend
a) mindestens ein ethylenisch ungesättigtes, säuregruppentragendes Monomer, das zumindest teilweise neutralisiert ist,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
umfassend die Schritte
i) kontinuierliche Vorneutralisation des Monomeren unter Kühlung und teilweiser Rückführung des vorneutralisierten Monomeren in die Vorneutralisation,
ii) kontinuierliche Erwärmung des vorneutralisierten Monomeren aus Schritt i) mittels eines Wärmetauschers,
iii) kontinuierliche Nachneutralisation des erwärmten Monomeren aus Schritt ii),
iv) Zusatz des Vernetzers zur Monomerlösung aus Schritt iii) und
v) Zusatz des Initiators zur Monomerlösung aus Schritt iv),
wobei die Monomerlösung oder -suspension vor dem Eintritt in den Polymerisationsreaktor eine Temperatur von 70 bis 99°C aufweist und die Temperatur des im Wärmetauscher verwendeten Heizmediums in Schritt ii) weniger als 160°C beträgt.

2. Verfahren nach Anspruch 1, **dadurch gekennzeichnet, dass** das vorneutralisierte Monomer in Schritt i) auf eine Temperatur von weniger als 45°C gekühlt wird.

3. Verfahren nach Anspruch 1 oder 2, **dadurch gekennzeichnet, dass** das vorneutralisierte Monomer in Schritt i) auf eine Temperatur von weniger als 40°C gekühlt wird.

4. Verfahren nach einem der Ansprüche 1 bis 3, **dadurch gekennzeichnet, dass** das vorneutralisierte Monomer in Schritt i) auf eine Temperatur von weniger als 35°C gekühlt wird.

5. Verfahren nach einem der Ansprüche 1 bis 4, **dadurch gekennzeichnet, dass** zwischen 40 und 98% des vorneutralisierten Monomeren in Schritt i) rückgeführt werden.

6. Verfahren nach einem der Ansprüche 1 bis 5, **dadurch gekennzeichnet, dass** zwischen 60 und 95% des vorneutralisierten Monomeren in Schritt i) rückgeführt werden.

7. Verfahren nach einem der Ansprüche 1 bis 6, **dadurch gekennzeichnet, dass** zwischen 80 und 90% des vorneutralisierten Monomeren in Schritt i) rückgeführt werden.

8. Verfahren nach einem der Ansprüche 1 bis 7, **dadurch gekennzeichnet, dass** die Temperatur des im Wärmetauscher verwendeten Heizmediums in Schritt ii) weniger als 140°C beträgt.

9. Verfahren nach einem der Ansprüche 1 bis 8, **dadurch gekennzeichnet, dass** die Temperatur des im Wärmetauscher verwendeten Heizmediums in Schritt ii) weniger als 120°C beträgt.

10. Verfahren nach einem der Ansprüche 1 bis 9, **dadurch gekennzeichnet, dass** die Temperatur des im Wärmetauscher verwendeten Heizmediums in Schritt ii) weniger als 110°C beträgt.

11. Verfahren nach einem der Ansprüche 1 bis 10, **dadurch gekennzeichnet, dass** der Neutralisationsgrad in Schritt iii) um 5 bis 45 mol-% erhöht wird.

12. Verfahren nach einem der Ansprüche 1 bis 11, **dadurch gekennzeichnet, dass** der Neutralisationsgrad in Schritt iii) um 10 bis 35 mol-% erhöht wird.

13. Verfahren nach einem der Ansprüche 1 bis 12, **dadurch gekennzeichnet, dass** der Neutralisationsgrad in Schritt iii) um 15 bis 25 mol-% erhöht wird.

14. Verfahren nach einem der Ansprüche 1 bis 13, **dadurch gekennzeichnet, dass** das Monomer a) Acrylsäure ist.

15. Verfahren nach einem der Ansprüche 1 bis 14, **dadurch gekennzeichnet, dass** die Superabsorberpartikel oberflächennachvernetzt werden.

## Claims

1. A process for producing superabsorbent particles by polymerizing a monomer solution or suspension comprising
a) at least one ethylenically unsaturated monomer which bears acid groups and is at least partly neutralized,
b) at least one crosslinker,
c) at least one initiator,
d) optionally one or more ethylenically unsaturated monomers copolymerizable with the monomers mentioned under a) and
e) optionally one or more water-soluble polymers, comprising the steps of
i) continuously preneutralizing the monomer while cooling and partly recycling the preneutralized monomer into the preneutralization,
ii) continuously heating the preneutralized monomer from step i) by means of a heat exchanger,
iii) continuously postneutralizing the heated monomer from step ii),
iv) adding the crosslinker to the monomer solution from step iii) and
v) adding the initiator to the monomer solution from step iv),
wherein the monomer solution or suspension is at a temperature of 70 to 99°C prior to entry into the polymerization reactor and the temperature of the heating medium used in the heat exchanger in step ii) is less than 160°C.

2. The process according to claim 1, wherein the preneutralized monomer is cooled in step i) to a temperature of less than 45°C.

3. The process according to claim 1 or 2, wherein the preneutralized monomer is cooled in step i) to a temperature of less than 40°C.

4. The process according to any of claims 1 to 3, wherein the preneutralized monomer is cooled in step i) to a temperature of less than 35°C.

5. The process according to any of claims 1 to 4, wherein between 40% and 98% of the preneutralized monomer is recycled into step i).

6. The process according to any of claims 1 to 5, wherein between 60% and 95% of the preneutralized monomer is recycled into step i).

7. The process according to any of claims 1 to 6, wherein between 80% and 90% of the preneutralized monomer is recycled into step i).

8. The process according to any of claims 1 to 7, wherein the temperature of the heating medium used in the heat exchanger in step ii) is less than 140°C.

9. The process according to any of claims 1 to 8, wherein the temperature of the heating medium used in the heat exchanger in step ii) is less than 120°C.

10. The process according to any of claims 1 to 9, wherein the temperature of the heating medium used in the heat exchanger in step ii) is less than 110°C.

11. The process according to any of claims 1 to 10, wherein the neutralization level is increased in step iii) by 5 to 45 mol%.

12. The process according to any of claims 1 to 11, wherein the neutralization level is increased in step iii) by 10 to 35 mol%.

13. The process according to any of claims 1 to 12, wherein the neutralization level is increased in step iii) by 15 to 25 mol%.

14. The process according to any of claims 1 to 13, wherein monomer a) is acrylic acid.

15. The process according to any of claims 1 to 14, wherein the superabsorbent particles are surface postcrosslinked.

## Revendications

1. Procédé pour la production de particules superabsorbantes, par polymérisation d'une solution ou suspension de monomères contenant
a) au moins un monomère à insaturation éthylénique, portant des groupes acides, qui est au moins partiellement neutralisé,
b) au moins un agent de réticulation,
c) au moins un amorceur,
d) en option un ou plusieurs monomères à insaturation éthylénique copolymérisables avec les monomères cités en a) et
e) en option un ou plusieurs polymères hydrosolubles, comprenant les étapes
i) la pré-neutralisation continue du monomère avec refroidissement et recyclage partiel du monomère pré-neutralisé vers la pré-neutralisation,
ii) le chauffage continu du monomère pré-neutralisé de l'étape i) au moyen d'un échangeur de chaleur,
iii) la post-neutralisation continue du monomère chauffé de l'étape ii),
iv) l'ajout de l'agent de réticulation à la solution de monomères de l'étape iii) ; et
v) l'ajout de l'initiateur à la solution de monomères de l'étape iv),
la solution ou la suspension de monomères présentant une température de 70 à 99 °C avant l'entrée dans le réacteur de polymérisation et la température du milieu chauffant utilisé dans l'échangeur de chaleur dans l'étape ii) étant inférieure à 160 °C.

2. Procédé selon la revendication 1, **caractérisé en ce que** le monomère pré-neutralisé est refroidi à une température inférieure à 45 °C à l'étape i).

3. Procédé selon la revendication 1 ou 2, **caractérisé en ce que** le monomère pré-neutralisé est refroidi à une température inférieure à 40 °C à l'étape i).

4. Procédé selon l'une quelconque des revendications 1 à 3, **caractérisé en ce que** le monomère pré-neutralisé est refroidi à une température inférieure à 35 °C à l'étape i).

5. Procédé selon l'une quelconque des revendications 1 à 4, **caractérisé en ce qu'**entre 40 et 98 % du monomère pré-neutralisé est recyclé dans l'étape i).

6. Procédé selon l'une quelconque des revendications 1 à 5, **caractérisé en ce qu'**entre 60 et 95 % du monomère pré-neutralisé est recyclé dans l'étape i).

7. Procédé selon l'une quelconque des revendications 1 à 6, **caractérisé en ce qu'**entre 80 et 90 % du monomère pré-neutralisé est recyclé dans l'étape i).

8. Procédé selon l'une quelconque des revendications 1 à 7, **caractérisé en ce que** la température du milieu chauffant utilisé dans l'échangeur de chaleur à l'étape ii) est inférieure à 140 °C.

9. Procédé selon l'une quelconque des revendications 1 à 8, **caractérisé en ce que** la température du milieu chauffant utilisé dans l'échangeur de chaleur à l'étape ii) est inférieure à 120 °C.

10. Procédé selon l'une quelconque des revendications 1 à 9, **caractérisé en ce que** la température du milieu chauffant utilisé dans l'échangeur de chaleur à l'étape ii) est inférieure à 110 °C.

11. Procédé selon l'une quelconque des revendications 1 à 10, **caractérisé en ce que** le degré de neutralisation est augmenté de 5 à 45 % en moles à l'étape iii).

12. Procédé selon l'une quelconque des revendications 1 à 11, **caractérisé en ce que** le degré de neutralisation est augmenté de 10 à 35 % en moles à l'étape iii).

13. Procédé selon l'une quelconque des revendications 1 à 12, **caractérisé en ce que** le degré de neutralisation est augmenté de 15 à 25 % en moles à l'étape iii).

14. Procédé selon l'une quelconque des revendications 1 à 13, **caractérisé en ce que** le monomère a) est l'acide acrylique.

15. Procédé selon l'une quelconque des revendications 1 à 14, **caractérisé en ce que** les particules superabsorbantes sont post-réticulées en surface.
